(19) Europäisches Patentamt
European Patent Office
Office européen des brevets

(11) Veröffentlichungsnummer: **0 021 241**
**B1**

(12) # EUROPÄISCHE PATENTSCHRIFT

(45) Veröffentlichungstag der Patentschrift: **22.06.83**

(21) Anmeldenummer: **80103236.8**

(22) Anmeldetag: **11.06.80**

(51) Int. Cl.³: **C 07 C 27/06**, C 07 C 31/08, C 07 C 47/06, C 07 C 53/08, C 07 C 1/04, C 07 C 11/04, C 07 C 11/06, C 07 C 11/08

(54) **Verfahren zur Herstellung von sauerstoffhaltigen Kohlenstoffverbindungen und Olefinen aus Synthesegas.**

(30) Priorität: **21.06.79 DE 2924962**

(43) Veröffentlichungstag der Anmeldung: **07.01.81 Patentblatt 81/01**

(45) Bekanntmachung des Hinweises auf die Patenterteilung: **22.06.83 Patentblatt 83/25**

(84) Benannte Vertragsstaaten: **BE DE FR GB IT NL**

(56) Entgegenhaltungen:
**EP A 0 004 653**
**EP A 0 004 656**
**EP A 0 018 763**
**FR A 2 277 805**
**FR A 2 326 397**
**FR A 2 326 399**
**FR A 2 391 982**

(73) Patentinhaber: **HOECHST AKTIENGESELLSCHAFT**
**Postfach 80 03 20**
**D-6230 Frankfurt/Main 80 (DE)**

(72) Erfinder: **Wunder, Friedrich, Dr.**
**Jahnstrasse 46**
**D-6093 Flörsheim am Main (DE)**
Erfinder: **Arpe, Hans-Jürgen, Dr.**
**de-Ridder-Weg 10**
**D-6230 Frankfurt am Main 80 (DE)**
Erfinder: **Leupold, Ernst Ingo, Dr.**
**Am Zäunefeld 15**
**D-6392 Neu-Anspach (DE)**
Erfinder: **Schmidt, Hans-Joachim, Dr.**
**Am Burgenblick 6**
**D-6240 Königstein/Taunus (DE)**
Erfinder: **Hachenberg, Horst, Dr.**
**Mohnweg 1**
**D-6229 Walluf (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

# Verfahren zur Herstellung von sauerstoffhaltigen Kohlenstoffverbindungen und Olefinen aus Synthesegas

Die Erfindung betrifft ein Verfahren zur Herstellung von Gemischen aus sauerstoffhaltigen $C_2$-Verbindungen und niedermolekularen Olefinen. Sie betrifft insbesondere die Herstellung von Gemischen aus Essigsäure, Acetaldehyd, Ethanol, Ethylen und Propylen durch Umsetzung von Kohlenmonoxid mit Wasserstoff in der Gasphase.

Es sind bereits zahlreiche Verfahren bekannt, in denen die Gasphasenumsetzung von Synthesegas, d. h. von Mischungen des Kohlenmonoxids und Wasserstoffs, an Eisen- oder Kobalthaltigen Katalysatoren zu Gemischen aus sauerstoffhaltigen Kohlenstoffverbindungen und gesättigten oder ungesättigten Kohlenwasserstoffen beschrieben wird. Diese allgemein als Fischer-Tropsch-Verfahren bekannte Umsetzung des Synthesegases ist jedoch wenig selektiv und führt zu Gemischen mit einer breiten, wenig spezifischen Produktverteilung, wobei die Einzelkomponenten bis zu 20 oder mehr Kohlenstoffatome enthalten können. Der Zusatz von Alkali, insbesondere Kaliumcarbonat bzw. Kaliumoxid, bewirkt bei diesen Katalysatoren zwar eine verminderte Methan- und erhöhte Olefinbildung, führt andererseits aber zu einem verstärkten Kettenwachstum, d. h. die Bildung höhermolekularer Verbindungen wird durch den Alkalizusatz begünstigt (vgl. Büssemeier et al, Hydrocarbon Processing, Nov. 1976, Seite 161).

Aus der deutschen Offenlegungsschrift 2 507 647 ist weiter bekannt, daß an überwiegend Mangan enthaltenden Katalysatoren bevorzugt Olefine mit zwei bis vier Kohlenstoffatomen im Molekül neben sauerstoffhaltigen Verbindungen entstehen können. Bei diesem Verfahren wird jedoch ein großer Teil des Kohlenmonoxids zu Kohlendioxid umgesetzt, außerdem ist der Anteil an sauerstoffhaltigen Verbindungen im Reaktionsgemisch sehr gering.

In neuerer Zeit sind weiterhin zahlreiche Verfahren bekannt geworden, die die Gasphasenumsetzung von Synthesegas an Rhodium enthaltenden Katalysatoren zum Gegenstand haben und die in hoher Selektivität zu sauerstoffhaltigen Verbindungen mit vorzugsweise zwei Kohlenstoffatomen im Molekül führen. Solche Verfahren sind beispielsweise aus DE-AS 2 503 233, DE-AS 2 503 204, DE-OS 2 628 463 und der US-Patentschrift 4 096 164 bekannt bzw. wurden in den deutschen Patentanmeldungen P 28 14 365.9, P 28 14 427.6, P 28 25 495.7, P 28 25 598.3, P 28 50 110.2 und P 28 50 201.4 vorgeschlagen.

Diese auf Basis von Rhodium als katalytisch aktiver Komponente beruhenden Verfahren geben neben sauerstoffhaltigen Produkten mit vorzugsweise zwei Kohlenstoffatomen im Molekül im wesentlichen noch Kohlendioxid, Methan und nur geringe Mengen anderer gesättigter bzw. ungesättigter Kohlenwasserstoffe. So reagieren beispielsweise nach M.M. Bhasin et al (J. of Catalysis 54, 120 (1978)) bei der Umsetzung von Synthesegas an einem 2,5 Gew.-% Rhodium enthaltenden Katalysator bei 300 °C und 70 bar nur 3,4 % des umgesetzten Kohlenmonoxids zu gesättigten und ungesättigten Kohlenwasserstoffen mit zwei oder mehr Kohlenstoffatomen, 43,1 % des Kohlenmonoxids zu sauerstoffhaltigen $C_2$-Verbindungen und 52 % zu Methan.

Durch Verwendung von Katalysatoren, die neben Rhodium noch Promotoren wie Magnesium oder Mangan enthalten, lassen sich die sauerstoffhaltigen Verbindungen wie Essigsäure, Acetaldehyd und Ethanol in verbesserter Selektivität gewinnen. Wenn auch in vielen Fällen die möglichst selektive Herstellung dieser sauerstoffhaltigen Verbindungen im Vordergrund steht, so kann doch die gleichzeitige Gewinnung niedermolekularer Olefine, die technisch wichtige Basisprodukte der chemischen Industrie sind, von Bedeutung sein, insbesondere dann, wenn mit einer vermehrten Olefinbildung eine geringere Umsetzung zu Methan als Nebenprodukt verbunden ist.

Es wurde nun gefunden, daß man Gemische aus sauerstoffhaltigen $C_2$-Verbindungen und einem hohen Anteil niedermolekularer Olefine erhält, wenn man Katalysatoren verwendet, die neben Rhodium und gegebenenfalls Promotoren noch 0,1 bis 5,0 Gew.-% Natrium oder Kalium, aufgebracht durch Imprägnierung, enthalten. Außerdem wurde gefunden, daß durch den Zusatz der Alkaliverbindungen die Aktivität der Katalysatoren gesteigert und die Selektivität zu Methan verringert wird.

Gegenstand der Erfindung ist daher ein Verfahren zur Herstellung von Gemischen aus Essigsäure, Acetaldehyd, Ethanol und Olefinen mit zwei bis vier Kohlenstoffatomen im Molverhältnis der genannten sauerstoffhaltigen Verbindungen zu den Olefinen von 1 : 1 bis 2,5 : 1 durch Umsetzung von Kohlenmonoxid und Wasserstoff in Gegenwart von Rhodium und gegebenenfalls Promotoren enthaltenden Trägerkatalysatoren in der Gasphase bei Temperaturen zwischen 150 und 350 °C und Drücken zwischen 1 und 300 bar, dadurch gekennzeichnet, daß die Katalysatoren 0,1 bis 5,0 Gew.-% Natrium oder Kalium enthalten, aufgebracht durch Imprägnierung mit entsprechenden Oxiden, Hydroxiden, Salzen oder Komplexverbindungen, wobei Doppelsalze des Rhodiums oder Magnesiums mit Natrium- oder Kaliumhalogeniden sowie Kalium-Rhodium-Cluster-Komplexe ausgenommen sind.

Der Befund, daß Alkali-Ionen in geringen Konzentrationen die Bildung von Olefinen begünstigen, die Umsetzung des Synthesegases zu Methan verringern und zusätzlich die Gesamtaktivität der Katalysatoren erhöhen, war überraschend und nicht vorauszusehen.

Bei dem erfindungsgemäßen Verfahren entstehen als Olefine hauptsächlich Ethylen und Propylen, neben geringen Mengen Butenen, und als sauerstoffhaltige Verbindungen Essigsäure, Acetaldehyd und Ethanol, sowie solche Produkte, die in einer Folgereaktion, beispielsweise durch Veresterung oder Kondensation, unter den Reaktionsbedingungen gebildet werden können, vor allem Ethylacetat und das Diethylacetal des Acetaldehyds.

Die Gesamtselektivität zu sauerstoffhaltigen Produkten und Olefinen liegt im allgemeinen zwischen 70 und 90 %, bezogen auf umgesetztes Kohlenmonoxid. Restliches Kohlenmonoxid wird zu Alkanen einschließlich Methan, zu Kohlendioxid und in geringen Mengen zu sauerstoffhaltigen Verbindungen mit drei oder mehr Kohlenstoffatomen umgesetzt.

Das Molverhältnis von sauerstoffhaltigen $C_2$-Verbindungen zu Olefinen liegt zwischen 1 : 1 und 2,5 : 1, wobei als Molverhältnis das Verhältnis der Molsumme der sauerstoffhaltigen $C_2$-Verbindungen, also Essigsäure, Acetaldehyd und Ethanol, zur Molsumme der Olefine mit 2 bis 4 C-Atomen zu verstehen ist.

Als Träger können handelsübliche Trägermaterialien mit unterschiedlichen spezifischen Oberflächen verwendet werden. Allerdings werden Träger mit spezifischen Oberflächen von 50 bis 1 000 $m^2/g$ bevorzugt. Geeignet sind z. B. Kieselsäure, natürliche oder synthetische Silikate von Elementen der II. bis VIII. Gruppe des Periodischen Systems (also beispielsweise die Silikate des Magnesiums, Calciums, Aluminiums, der Seltenen Erden, des Titans, Zirkons, Mangans), ferner Aluminiumoxid, Titandioxid, Zirkondioxid, Thoriumdioxid, Zeolithe und Spinelle.

Rhodium kann auf dem Träger in metallischer Form oder auch in einer Wertigkeitsstufe unter drei, also als Komplexverbindung des nullwertigen Rhodiums oder als Salz oder Komplexverbindung des ein- oder zweiwertigen Rhodiums vorliegen. Man kann dabei von Salzen oder Komplexverbindungen des Rhodiums beliebiger Wertigkeitsstufe ausgehen und gegebenenfalls eine Reduktionsstufe anschließen, wie sie weiter unten beschrieben ist. Geeignete Verbindungen des Rhodiums sind z. B. Chloride, Bromide, Jodide, Nitrate oder Carboxylate.

Geeignet sind ferner Komplexverbindungen, die neben Rhodium und Halogen noch komplexbildende Liganden, wie Trialkylphosphin, Triarylphosphin, Ethylendiamin, Pyridin, Kohlenmonoxid, Olefine oder Wasser enthalten, also z. B. Tris-triphenylphosphin-rhodium-I-chlorid, -bromid oder -jodid, Tris-triphenylphosphin-rhodium-III-chlorid, Dichlor-bis-ethylendiamin-rhodium-I-chlorid, Tris-ethylendiamin-rhodium-III-chlorid, Bis-tri-otolyl-phosphin-rhodium-II-chlorid, Carbonyl-bis-triphenyl-phosphin-rhodium-I-bromid oder Dicäsium-carbonyl-pentachloro-rhodat-III. Darüber hinaus kommen auch solche Verbindungen des Rhodiums in Betracht, in denen es ionogen oder komplex an einen Träger gebunden ist. Beispiele hierfür sind die mit Rhodiumhalogeniden ausgetauschten Zeolithe und Ionenaustauscher.

Als Alkaliverbindungen werden die Oxide, Hydroxide, Salze oder Komplexverbindungen des Natriums oder Kaliums oder deren Gemische verwendet, also beispielsweise die Oxide, Hydroxide, Carbonate, Chloride, Bromide, Jodide, Nitrate, Acetate, Silikate und/oder Aluminate der Alkalimetalle. Besonders bevorzugt sind die Natriumverbindungen. Ausgenommen sind die Doppelsalze des Rhodiums oder Magnesiums mit Natrium- oder Kaliumhalogeniden sowie Kalium-Rhodium-Clusterkomplexe.

Die Katalysatoren enthalten neben Rhodium und Alkaliverbindungen vorzugsweise noch Promotoren oder Aktivatoren. Als solche sind besonders geeignet die Kombination Magnesium/Halogenidionen sowie Mangan. Gegebenenfalls können die Katalysatoren aber auch solche Stoffe enthalten, die die Selektivität zu den einzelnen sauerstoffhaltigen Produkten beeinflussen, wie Eisen, Zirkon, Hafnium, Lanthan, Platin, Quecksilber, Molybdän, Wolfram. Die genannten Elemente, die als Promotoren wirken oder die Selektivität beeinflussen, können als einfache anorganische oder organische Verbindungen vorliegen, wie z. B. als Chloride, Bromide, Nitrate, Carbonate, Oxide, Hydroxide, Silicate oder Acetate. Geeignet sind auch Komplexverbindungen dieser Elemente mit anorganischen oder organischen Liganden, wie z. B.

Trinatriumhexacyanomanganat-(III), Dikaliumhexacyanoferrat-(II), ferner Chlorokomplexverbindungen der genannten Elemente mit Rhodium der allgemeinen Formel $Me_m[RhCl_6]_n$, wobei Me für eines der genannten Metalle steht, beispielsweise $Mg_3[RhCl_6]_2$. Die in Kombination mit Magnesium verwendeten Halogenidionen können die Chloride, Bromide oder Jodide sein. Das Halogenid kann als Rhodium-, Alkali- oder Magnesiumverbindung aufgebracht werden ; geeignete Verbindungen sind bereits erwähnt worden.

Man kann aber auch halogenfreie Magnesiumverbindungen, z. B. die Acetate oder Nitrate einsetzen und durch anschließende Behandlung mit Halogenwasserstoff oder Imprägnierung mit einem Metallhalogenid die Halogenidionen auf den Träger aufbringen. Man kann auch mit einer Halogen enthaltenden organischen Verbindung (wie z. B. 1,1-Dichloräthan), aus der Halogen freizusetzen ist, den für die selektive Synthesegasumsetzung erforderlichen Halogengehalt des Katalysators nach der Imprägnierung mit der Magnesiumverbindung einstellen.

Als Lösungsmittel für die aktiven Komponenten eigenen sich z. B. Wasser und wasserfreie oder wasserhaltige Carbonsäuren, insbesondere Wasser und Essigsäure.

Zum Aufbau des Katalysators wird der Träger mit der Rhodiumverbindung, der Alkaliverbindung und ggf. den Promotoren bzw. den Zusatzstoffen, die die Selektivität beeinflussen, gleichzeitig oder in aufeinanderfolgenden Stufen in beliebiger Reihenfolge getränkt.

Eine besonders bevorzugte Form der Katalysatorherstellung besteht darin, den Träger mit der Lösung der Alkaliverbindung und des Promotors zu tränken, zu trocknen, anschließend bei Temperaturen zwischen 400 und 1 200 °C zu sintern und danach die Rhodiumverbindung aufzubringen.

Das Alkalimetall und die Promotoren können aber auch in eine Gerüstsubstanz eingebaut werden, beispielsweise in eine Silikat oder Aluminiumoxid enthaltende Trägersubstanz, wie Kieselsäure, Aluminiumoxid oder Aluminiumsilikat. Eine weitere vorteilhafte Möglichkeit besteht darin, das Alkalimetall oder die Promotoren mittels Ionenaustausch an Kationenaustauscher zu binden, die auch als Träger für das Rhodium geeignet und unter den Versuchsbedingungen beständig sind, beispielsweise die als Molsiebe bekannten natürlichen oder synthetischen Aluminiumsilikate.

Vor Verwendung des Katalysators für die Synthesegasumsetzung muß noch reduziert werden, wenn die Umsetzung an metallischem Rhodium erfolgen soll, oder wenn für die Katalysatorherstellung dreiwertiges Rhodium verwendet wurde. Die Reduktion kann im Reaktor selbst oder in einer getrennten Apparatur durchgeführt werden. Als Reduktionsmittel kommen beispielsweise Wasserstoff, Kohlenmonoxid, Methanol oder Aceton in Betracht. Reduktionstemperaturen oberhalb 300°, vorzugsweise zwischen 350 und 550 °C führen zu metallischem Rhodium, Reduktionstemperaturen unter 300 °C, vorzugsweise zwischen 100 und 275 °C, zu einer niederen (nicht-metallischen) Wertigkeitsstufe des Rhodiums.

Vielfach ist es zweckmäßig, die Reduktion nicht mit dem unverdünnten Reduktionsmittel vorzunehmen, sondern es mit einem zusätzlichen Anteil an Inertgas, wie z. B. Stickstoff oder Kohlendioxid, zu verdünnen.

Die Konzentration an Rhodium, den Alkalimetallen und gegebenenfalls den Promotoren kann in weiten Grenzen variiert werden. Im allgemeinen liegen die Werte bezogen auf die Metalle zwischen 0,1 und 15 Gew.-% für Rhodium, zwischen 0,1 und 5,0 Gew.-% für die Alkalimetalle und zwischen 0,1 und 20 Gew.-% für die Promotoren. Bei Einsatz der Kombination Magnesium/Halogenidionen werden 0,1-10 Gew.-% Magnesium und 0,1-10 Gew.-% Halogenidionen verwendet. Die Alkalimetallkonzentration beeinflußt die Selektivität zu den Olefinen, und zwar nimmt mit zunehmender Konzentration die Selektivität zu den Olefinen zu.

Zur Durchführung des erfindungsgemäßen Verfahrens werden Gasgemische, die ganz oder zu einem überwiegenden Teil aus Kohlenmonoxid und Wasserstoff bestehen und daneben gegebenenfalls noch andere Komponenten wie Stickstoff, Argon, Kohlendioxid oder Methan enthalten können, über den Katalysator geleitet. Das molare Verhältnis von Kohlenmonoxid zu Wasserstoff kann dabei in weiten Grenzen variiert werden. Bevorzugt sind Molverhältnisse zwischen 5 : 1 und 1 : 5 und besonders zwischen 3 : 1 und 1 : 3. Die Reaktionstemperaturen liegen im allgemeinen zwischen 150 und 350 °C, vorzugsweise zwischen 200 und 350 °C, und die Reaktionsdrücke zwischen 1 und 300 bar, vorzugsweise zwischen 10 und 200 bar.

Zweckmäßig ist es, Temperatur und Druck so aufeinander abzustimmen, daß eine hohe Selektivität zu den sauerstoffhaltigen Verbindungen gewährleistet ist und die bei höheren Temperaturen begünstigte exotherme Bildung von Methan gering bleibt. Man wird deshalb hohe Drücke und möglichst niedrige Temperaturen bevorzugen. Der Umsatz an Kohlenmonoxid sollte dabei im allgemeinen nicht über 50 % liegen, da höhere Umsätze leicht zu vermehrter Nebenproduktbildung führen können, wobei neben Methan und Kohlendioxid auch höhermolekulare flüssige Kohlenwasserstoffe und sauerstoffhaltige Produkte auftreten können.

Für die Verfahrensdurchführung können die herkömmlichen Festbettreaktoren verwendet werden, wobei es zur besseren Wärmeabführung vorteilhaft ist, die Katalysatorschichtdicke gering zu halten. Ferner sind auch Reaktoren mit bewegtem Katalysatorbett oder Wirbelbettreaktoren geeignet.

Eine besonders bevorzugte Ausführungsform der Erfindung besteht darin, die Umsetzung in einer Kreisgasapparatur durchzuführen, in der nach Abtrennung der kondensierbaren Reaktionsprodukte das nicht umgesetzte Gasgemisch wieder in den Reaktor zurückgeführt wird.

Diese Verfahrensweise ist besonders wirtschaftlich und ermöglicht durch Verdünnung des Frischgases mit dem im Kreisgas zurückgeführten wasserstoffärmeren Restgas höhere Reaktionstemperaturen und damit höhere Raumzeitausbeuten bei unveränderten Selektivitäten. Als Kreisgasapparaturen können dabei solche mit innerem oder äußerem Gasumlauf in Betracht kommen.

Das Wesen der Erfindung soll in folgenden Beispielen erläutert werden, wobei die Beispiele in keiner Weise einschränkend sein sollen.

(Vergleichsbeispiele 1-5) Versuchsreihe A

(Verwendung handelsüblicher Träger mit bereits vorhandenem unterschiedlichem Natriumgehalt).

Je 52 g (120 ml) der in der Tabelle 1 aufgeführten Kieselsäureträger werden mit einer Lösung von 1,08 g Magnesiumchloridhexahydrat in 66 g Wasser bei Raumtemperatur getränkt, dann 2 Stunden bei 80 °C und 2 Stunden bei 150 °C getrocknet. Anschließend wird 30 Minuten bei 800 °C gesintert. Der gesinterte Träger wird danach mit einer Lösung von 4,0 g Rhodium-III-chlorid · $xH_2O$ (37,4 Gew.-% Rh) in 66 g Wasser bei Raumtemperatur getränkt und in gleicher Weise wie oben beschrieben getrocknet. Die Katalysatoren werden dann in einem Glasgefäß durch 3-stündiges Überleiten von 30 Nl/h Wasserstoff bei 225-275 °C unter Normaldruck reduziert. Sie enthalten 2,7 Gew.-% Rh und 0,24 Gew.-% Mg.

100 ml des Katalysators werden in ein Reaktionsrohr aus rostfreiem Stahl von 810 mm Länge und 16 mm innerem Durchmesser gegeben, das mit einem koaxial angebrachten Thermometerrohr von 6 mm äußerem Durchmesser versehen ist. Die Reaktortemperatur wird mit einem Salzbad eingestellt.

Über den Katalysator leitet man bei 20 bar und 275 °C Innentemperatur stündlich 70 Nl eines Gemisches von Kohlenmonoxid und Wasserstoff im Volumenverhältnis 1 : 1. Das Reaktionsgemisch wird gekühlt und die nicht kondensierbaren Komponenten werden entspannt. Die gasförmigen Komponenten und die kondensierten Reaktionsprodukte werden gaschromatographisch bestimmt. Es werden die in Tabelle 1 zusammengefaßten Ergebnisse erhalten.

Tabelle 1

Reaktionsbedingungen : 20 bar, 275 °C, Einsatzgas 70 Nl/h, $CO : H_2 = 1$.
Katalysatorvolumen 0,1 l, Zusammensetzung : 2,7 % Rh, 0,24 % Mg auf $SiO_2$.
AcOH = Essigsäure, AcH = Acetaldehyd, EtOH = Ethanol.

| Vergl. Beisp. Nr. | % Na [1] [5] | CO-Umsatz (Mol.-%) | RZA [2] | Selektivität [3] zu AcOH | AcH | EtOH | $C_2H_4$ | $C_3H_6$ | $C_4H_8$ | $CH_4$ | Molverhältnis [4] |
|---|---|---|---|---|---|---|---|---|---|---|---|
| 1 | 0,04 | 28,0 | 36 | 15 | 12 | 3 | 0,6 | 2,2 | 0,2 | 53 | 13,8 |
| 2 | 0,07 | 26,1 | 41 | 19 | 14 | 2,5 | 2,6 | 10,7 | 0,7 | 38 | 3,5 |
| 3 | 0,12 | 28,4 | 47 | 18 | 16,5 | 3,5 | 4,9 | 17,6 | 0,6 | 25 | 2,2 |
| 4 | 0,18 | 29,8 | 57 | 22,5 | 17 | 4 | 6,3 | 21,0 | 0,7 | 15 | 2,1 |
| 5 | 0,25 | 30,8 | 62 | 24 | 19,2 | 3 | 7,5 | 25,3 | 1,2 | 8 | 1,85 |

1) Na-Gehalt im handelsüblichen Träger.
2) Raumzeitausbeute (in g) sauerstoffhaltige $C_2$-Produkte pro Liter Katalysator und Stunde.
3) In Mol. % vom umgesetzten CO ; Ethylacetat und Diethylacetal des Acetaldehyds sind in Essigsäure, Acetaldehyd bzw. Ethanol umgerechnet.
4) Molsumme AcOH, AcH, EtOH ;
5) Molsumme $C_2H_4$, $C_3H_6$, $C_4H_9$.

| Vergl. Beisp. | 1 | 2 | 3 | 4 | 5 |
|---|---|---|---|---|---|
| BET Oberfl. ($m^2/g$) | 316 | 307 | 320 | 300 | 270 |
| Porenvol. (ml/g) | 1.51 | 1.25 | 1.15 | 1.25 | 1.22 |

Versuchsreihe B

(Verwendung von Trägern, die mit verschiedenen Alkalisalzen dotiert sind).

Die in Tabelle 2 angegebenen Mengen Alkalisalz, die jeweils 8,16 mmol der wasserfreien Verbindung entsprechen, werden in je 66 g Wasser gelöst. Mit diesen Lösungen werden je 52 g (120 ml) des in Vergleichsbeispiel 1 der Versuchsreihe A genannten Kieselsäureträgers getränkt. Nach dem Tränken wird 2 Stunden bei 80 °C und 2 Stunden bei 150 °C getrocknet und schließlich 30 Minuten bei 800 °C gesintert. Anschließend werden die Träger mit einer Lösung von je 1,08 g Magnesiumchlorid-hexahydrat in 66 g Wasser getränkt und in gleicher Weise, wie vorstehend beschrieben, getrocknet und gesintert. Dann werden die so vorbehandelten Träger noch mit einer Lösung von 4,0 g Rhodium-III-chlorid · $xH_2O$ (37,4 Gew.-% Rh) in 66 g Wasser getränkt, 2 Stunden bei 80 °C und 2 Stunden bei 150 °C getrocknet und zuletzt in einem Glasgefäß durch dreistündiges Überleiten von 30 Nl/h Wasserstoff bei 225-275 °C unter Normaldruck reduziert. Die Katalysatoren enthalten 2,7 Gew.-% Rh und 0,24 Gew.-% Mg.

Die Ausprüfung dieser Katalysatoren erfolgt in dem in Versuchsreihe A beschriebenen Reaktor unter den dort angegebenen Reaktionsbedingungen. Die Ergebnisse sind in Tabelle 2 zusammengefaßt.

Tabelle 2

Reaktionsbedingungen : 20 bar, 275 °C, Einsatzgas 70 Nl/h, $CO : H_2 = 1$.
Katalysatorvolumen 0,1 l, Zusammensetzung : 2,7 % Rh, 0,24 % Mg auf $SiO_2$.
AcOH = Essigsäure, AcH = Acetaldehyd, EtOH = Ethanol.

| Beisp. Nr. | Zusatz g Alkali-verbind. (wasserfrei) | % Alkali [1] | CO-Umsatz % | RZA [2] | Selektivität [3] AcOH | AcH | EtOH | $C_2H_4$ | $C_3H_6$ | $C_4H_8$ | $CH_4$ | Molverhältnis [4] |
|---|---|---|---|---|---|---|---|---|---|---|---|---|
| 1 | 0,670 NaOAc | 0,4 | 31,5 | 67,6 | 26,5 | 19 | 3 | 8 | 28,6 | 0,4 | 5 | 1,77 |
| 2 | 0,477 NaCl | 0,4 | 33,2 | 72,5 | 28 | 18 | 3 | 10,2 | 23,1 | 0,7 | 6 | 1,88 |
| 3 | 0,694 $NaNO_3$ | 0,4 | 29,5 | 46 | 17 | 12 | 7 | 9,6 | 21,9 | 0,5 | 12 | 1,47 |
| 4 | 0,538 LiOAc | 0,15 | 17,0 | 32 | 21 | 18 | 4 | 12,5 | 19,8 | 0,7 | 10 | 1,65 |

| Beisp. Nr. | Zusatz g Alkali-verbind. (wasserfrei) | % Alkali [1] | CO-Umsatz % | RZA [2] | AcOH | AcH | EtOH | $C_2H_4$ | $C_3H_6$ | $C_4H_8$ | $CH_4$ | Molverhältnis [4] |
|---|---|---|---|---|---|---|---|---|---|---|---|---|
| | | | | | Selektivität [3] | | | | | | | |
| 5 | 0,800 KOAc | 0,65 | 17,7 | 33 | 23 | 14 | 5 | 8,6 | 14,4 | 1,0 | 13 | 1,82 |
| 6 | 1,204 $RbNO_3$ | 1,36 | 12,5 | 20,5 | 18 | 16 | 4 | 10,3 | 18,9 | 0,8 | 11 | 1,63 |
| 7 | 1,374 CsCl | 2,04 | 10,4 | 15 | 18 | 12 | 3 | 9,5 | 16,9 | 0,6 | 14 | 1,56 |

1) Einschl. 0,04 % Na im handelsüblichen Träger ; die zugesetzte Alkalimenge entspricht je 8,16 mMol.
2) Raumzeitausbeute in g sauerstoffhaltige $C_2$-Produkte pro Liter Katalysator und Stunde.
3), 4) Siehe die entspr. Fußnoten unter Tabelle 1.

## Versuchsreihe C

(Kreisgasapparatur)

Die Apparatur besteht aus einem beheizten Reaktionsrohr von 1 m Länge und 24,4 mm innerem Durchmesser aus korrosionsbeständigem Stahl mit einer koaxial angebrachten Thermometerhülse von 12 mm äußerem Durchmesser, einem nachgeschalteten Kondensator, einer Vorlage für das Kondensat und einem Kompressor für die Rückführung eines Teils der nichtkondensierten Gase zum Reaktor (Kreisgas). Es werden jeweils 250 ml der unten beschriebenen Katalysatoren eingefüllt. Nach Spülen der Apparatur mit Stickstoff wird zunächst mit einem Synthesegas der Zusammensetzung 49 Vol.-% CO, 49 Vol.-% $H_2$, 1 Vol.-% $CO_2$, 1 Vol.-% $N_2$ (und geringe Mengen anderer Komponenten) ein Druck von 100 bar eingestellt und der Reaktor auf 275 °C aufgeheizt. Während des Aufheizens und im weiteren Versuchsverlauf werden stündlich 1 000 Nl Synthesegas der obigen Zusammensetzung über die Saugseite des Kompressors dem Kreisgas zugeführt und zusammen mit diesem über den Katalysator geleitet. Die Kreisgasmenge beträgt etwa 5 000 l/h. Das den Reaktor verlassende Gasgemisch wird in dem solegekühlten Kondensator auf etwa + 5 °C abgekühlt, und die kondensierten Anteile werden in der Vorlage aufgefangen. Das nicht kondensierte Restgas wird nach Vermischen mit frischem Synthesegas über den Kompressor wieder dem Reaktor zugeführt. Zur Aufrechterhaltung des Drucks und zur Ausschleusung der Olefine und der Nebenprodukte wird ein Teil des Restgases über ein Druckhalteventil abgeleitet. Nach dieser Methode werden das Vergleichsbeispiel 6 und die Beispiele 8-10 ausgeführt. Die Ergebnisse sind in Tabelle 3 zusammengestellt. Als Katalysator werden für Vergleichsbeispiel 6 250 ml des in Vergleichsbeispiel 1 genannten Katalysators eingesetzt. Für Beispiel 8 verwendet man 250 ml des in Beispiel 1 genannten Katalysators, und für Beispiel 9 verwendet man 250 ml des in Beispiel 2 genannten Katalysators.

Für Beispiel 10 werden 250 ml eines wie folgt hergestellten Katalysators eingesetzt :

120 g des in Vergleichsbeispiel 1 genannten Kieselsäureträgers mit einem Na-Gehalt von 0,04 Gew.-% werden mit einer Lösung von 2,49 g Magnesiumchlorid-hexahydrat und 1,55 g Natriumacetat in 152 g Wasser getränkt und 2 Stunden bei 80 °C, 2 Stunden bei 120 °C und 2 Stunden bei 150 °C getrocknet. Der Träger wird anschließend mit einer Lösung von 9,24 g Rhodium-III-chlorid (37,4 Gew.-% Rh) in 152 g Wasser getränkt und wieder in gleicher Weise, wie vorstehend beschrieben, getrocknet. Danach wird der Katalysator in einem Glasgefäß durch 3-stündiges Überleiten von 50 Nl/h Wasserstoff bei 225-275 °C unter Normaldruck reduziert. Der fertige Katalysator enthält 2,7 Gew.-% Rh, 0,24 Gew.-% Mg, 0,37 Gew.-% Na und 1,9 Gew.-% Cl.

### Tabelle 3

Kreisgasapparatur, 100 bar, 275 °C, Einsatzgas 1 000 Nl/h, CO : $H_2$ = 1.
Katalysatorvolumen 0,25 l, Zusammensetzung : 2,7 % Rh, 0,24 % Mg auf $SiO_2$.
AcOH = Essigsäure, AcH = Acetaldehyd, EtOH = Ethanol.

| Beisp. Nr. | % Alkali [1] | CO-Umsatz % | RZA [2] | AcOH | AcH | EtOH | $C_2H_4$ | $C_3H_6$ | $C_4H_8$ | $CH_4$ | Molverhältnis [4] |
|---|---|---|---|---|---|---|---|---|---|---|---|
| | | | | Selektivität [3] zu | | | | | | | |
| Vergl. Beisp. 6 | 0,04 | 21,6 | 380 | 42,1 | 27,6 | 4,8 | 2,3 | 4,4 | 0,4 | 8,7 | 13,7 |
| 8 | 0,4 | 39,2 | 452 | 31,5 | 18,3 | 3,7 | 9,6 | 24,1 | 0,8 | 3,8 | 2,05 |
| 9 | 0,4 | 33,9 | 428 | 37,4 | 12,3 | 2,1 | 11,5 | 23,6 | 1,0 | 4,1 | 1,86 |
| 10 | 0,37 | 34,6 | 410 | 32,6 | 15,5 | 1,5 | 12,2 | 25,1 | 1,1 | 3,2 | 1,68 |

1) Einschl. 0,04 % Na im handelsüblichen Träger.

2) Raumzeitausbeute in g sauerstoffhaltige $C_2$-Produkte pro Liter Katalysator und Stunde ; die angegebenen Werte sind Durchschnittswerte von 100 Stunden Betriebszeit.

3), 4) Siehe die entspr. Fußnoten zu Tabelle 1.

**Ansprüche**

1. Verfahren zur Herstellung von Gemischen aus Essigsäure, Acetaldehyd, Ethanol und Olefinen mit zwei bis vier Kohlenstoffatomen im Molverhältnis der genannten sauerstoffhaltigen Verbindungen zu den Olefinen von 1 : 1 bis 2,5 : 1 durch Umsetzung von Kohlenmonoxid und Wasserstoff in Gegenwart von Rhodium und gegebenenfalls Promotoren enthaltenden Trägerkatalysatoren in der Gasphase bei Temperaturen zwischen 150 und 350 °C und Drücken zwischen 1 und 300 bar, dadurch gekennzeichnet, daß die Katalysatoren 0,1 bis 5,0 Gew.-% Natrium oder Kalium enthalten, aufgebracht durch Imprägnierung mit entsprechenden Oxiden, Hydroxiden, Salzen oder Komplexverbindungen, wobei Doppelsalze des Rhodiums oder Magnesiums mit Natrium- oder Kaliumhalogeniden sowie Kalium-Rhodium-Clusterkomplexe ausgenommen sind.

2. Verfahren nach Anspruch 1, dadurch gekennzeichnet, daß als Alkalimetall Natrium verwendet wird.

3. Verfahren nach Anspruch 1 und 2, dadurch gekennzeichnet, daß man die mit Alkalimetall und gegebenenfalls mit Promotoren dotierten Träger vor der Imprägnierung mit Rhodium bei Temperaturen zwischen 400 und 1 200 °C sintert.

**Claims**

1. Process for the manufacture of mixtures of acetic acid, acetaldehyde, ethanol and olefins having two to four carbon atoms in a 1 : 1 to 2.5 : 1 molar ratio of the said oxygen-containing compounds to the olefins, by reacting carbon monoxide and hydrogen in the presence of catalysts containing rhodium and, if appropriate, promoters, in the gas phase at temperatures between 150 and 350 °C and under pressures between 1 and 300 bars, characterized in that the catalysts contain 0.1 to 5.0 % by weight of sodium or potassium which are applied by impregnation with corresponding oxides, hydroxides, salts or complex compounds with the exception of double salts of rhodium or magnesium with sodium or potassium halides and of potassium rhodium cluster complexes.

2. Process as claimed in claim 1, characterized in that sodium is used as the alkali metal.

3. Process as claimed in either of claims 1 or 2, characterized in that the supports, which have been doped with alkali metal and, if appropriate, with promoters, are sintered at temperatures between 400 and 1,200 °C before being impregnated with rhodium.

**Revendications**

1. Procédé de préparation de mélanges d'acide acétique, d'acétaldéhyde, d'éthanol et d'oléfines contenant de 2 à 4 atomes de carbone, mélanges dans lesquels le rapport molaire des composés oxygénés mentionnés aux oléfines est compris entre 1 : 1 et 2,5 : 1, par réaction de monoxyde de carbone et d'hydrogène en présence de catalyseurs avec supports contenant du rhodium et éventuellement des promoteurs, en phase gazeuse, à des températures comprises entre 150 et 350 °C et sous des pressions comprises entre 1 et 300 bar, procédé caractérisé en ce que les catalyseurs contiennent de 0,1 à 5,0 % en poids de sodium ou de potassium, appliqués par imprégnation avec des oxydes, hydroxydes, sels ou complexes correspondants, à l'exclusion de sels doubles du rhodium ou du magnésium avec des halogénures de sodium ou de potassium ainsi que des complexes dits « clusters » de potassium-rhodium.

2. Procédé selon la revendication 1, caractérisé en ce qu'on utilise le sodium comme métal alcalin.

3. Procédé selon l'une des revendications 1 et 2, caractérisé en ce que les supports, dopés par un métal alcalin et éventuellement par des promoteurs, sont frittés, avant l'imprégnation par le rhodium, à des températures comprises entre 400 et 1 200 °C.